# EUROPEAN PATENT SPECIFICATION

(11) **EP 0 410 207 B1**
(45) Date of publication and mention of the grant of the patent: **22.01.1997**
(21) Application number: 90113210.0
(22) Date of filing: 11.07.1990
(51) Int. Cl.: C12N 9/96

(54) **Stabilization of highly purified proteins**
Stabilisierung von hochgereinigten Proteinen
Stabilisation de protéines hautement purifiées

(30) Priority: 24.07.1989 US 384584
(43) Date of publication of application: 30.01.1991
(73) Proprietor: Bayer Corporation, Pittsburgh, PA 15219-2502 (US)
(72) Inventor: Coan, Michael H., El Cerrito, CA 94530 (US); Lee, Vivian W., El Sobrante, CA 94803 (US)
(74) Representative: Dänner, Klaus, Dr.

(56) References cited:
- EP-A- 0 306 968
- EP-A- 0 314 095
- WO-A-86/04486
- RÖMPP's CHEMIE LEXIKON, 8th edition, 1981; pp. 918-919

## Description

The present invention relates to the use of carbohydrates to stabilize Factor VIII:C and to compositions comprising Factor VIII:C, having a purity of at least 60 %, excipients and mannitol.

The prior art may be thought of as falling into three categories: (1) the stabilization of unpurified proteins, such as Factor VIII concentrates; (2) the stabilization of highly purified proteins; and (3) other uses of carbohydrates, such as dextran or sorbitol.

(1) Rock 4,359,463 discloses stabilization of Factor VIII activity in whole blood or blood plasma by addition of a calcium chelating anticoagulant such as acid citrate dextrose (ACD) or citrate phosphate dextrose (CPD). Heparin is also added.

Mathews et al. 4,743,680 disclose a method of purifying Factor VIII from plasma using a chromatography column exemplified by QAE-Sephadex, polyelectrolyte and aminohexyl-Sepharose resins. Sugars, polyhydric alcohols, and amino acids enhance the selective binding of proteins to these ion exchange chromatography media. In one example, 1M sorbitol is contained in a buffer which is added to the sample at the Al(OH)₃ absorption stage (Col. 5, 1. 63). Sugars, including sucrose, mannose and dextran can be used instead of a polyhydric alcohol (Col. 15, 1. 7).

Rasmussan et al. 4,650,858 disclose preparation of a high purity (50 u/mg) Factor VIII preparation using a PEG precipitation step which includes the use of "salting-in agent" such as an amino acid or a carbohydrate. Suitable carbohydrates may include sugar alcohols and oligosaccharides.

Thomas 4,089,944 discloses a method of improving the solubility of a Factor VIII composition by the addition of 0-5 grams of dextrose per 100 ml of reconstituted solution. Disaccharides and short chain oligosaccharides (dextrins) can also be used.

Winkelman 4,789,733 discloses the recovery of Factor VIII from plasma through precipitation with a sulphated polysaccharide, i.e. heparin. Dextran sulphate reportedly can also be used.

Schwinn et al. 4,297,344 disclose a heat treatment for plasma proteins including Factor VIII. An amino acid and a monosaccharide, oligosaccharide or sugar alcohol are added to an aqueous solution in order to stabilize the protein(s) against the heat. 20 to 60 w/w % sucrose is preferred.

Fernandes et al. 4,440,679 disclose heat treatment of plasma proteins (including Factor VIII) in the presence of a polyol as the sole stabilizing agent. The preferred "polyol" is sucrose; higher molecular weight polyols such as dextran, starch or glycogen are not preferred for use (Col. 6, 1. 44) with the disclosed process.

EP 077,870 discloses the heat treatment of Factor VIII in the presence of 10% (w/v) or more of at least one stabilizer selected from the group consisting of neutral amino acids, monosaccharides, oligosaccharides and sugar alcohols and 10% or more of an auxiliary stabilizer of a carboxylic acid with 3 - 10 carbon atoms.

Naito et al., 4,446,134 disclose heat treatment of Factor VIII concentrates with 10-60% (w/v) of a stabilizer selected from the group consisting of neutral amino acids, monosaccharides, oligosaccharides or sugar alcohols. A carboxylic acid is used as an auxiliary stabilizer.

Ng et al., Thromb. Res. 39:439-447 (1985) disclose a process for heat treating Factor VIII in solution which uses sucrose as a stabilizer.

Foster et al., Vox Sang. 55:81-89 (1988) discuss the stability of Factor VIII during the processing of Factor VIII:C from human plasma. Losses over the processing steps were minimized by the addition of calcium. The calcium prevented inactivation of Factor VIII:C caused by sodium citrate, which is added during the fractionation process. Various stabilizing agents (e.g. heparin, glycine and sugars) are referenced.

EP-A2-0 306 968 discloses the use of sorbitol, mannitol, dextrose, maltose, glycerol, human serum albumin or of a combination thereof as stabilizers for recombinant human Factor VIII:C.

EP-A1-0 314 095 discloses the stabilization of Factor VIII having a purity of more than 60 % in an aqueous solution comprising sodium and calcium chloride, histidine or lysine and optionally up to 10 % of mannitol, sucrose or maltose.

(2) With regard to highly purified, therapeutically active proteins, it is generally accepted that recombinant DNA derived proteins, regardless of whether from a bacterial, yeast or mammalian cell source, as well as monoclonal antibodies, must be purified to at least 99% purity if the product is intended for human use. Generally, human serum albumin is added to stabilize such purified proteins against denaturation or aggregation which may occur in a highly pure solution. The use of human serum albumin as a stabilizer has one drawback with regard to cost. In addition, albumin is not wholly satisfactory in conjunction with the use of therapeutic proteins, where the perceived benefit of using recombinant DNA derived proteins instead of plasma-derived proteins is greatly diminished when plasma-derived albumin is included in the final container formulation.

Plan et al. 3,992,367 disclose a purified albumin heated in the presence of caprylic acid, which is added for heat stabilization.

Vemura et al. 4,361,652 disclose the heat stabilization of plasma-derived plasminogen with inorganic salts such as NaCl.

Kwan 4,496,537 discloses the stabilization of alpha interferon preparations over the lyophilization step through the addition of glycine or alanine. 1-10 mg/ml albumin is also added as a stabilizer.

Mori et al. 4,505,893 disclose a purified plasminogen activator stabilized with 1-10 mg/ml of albumin. Murakimi et al. 4,552,760 disclose a method for stabilizing tissue plasminogen activator through the addition of gelatin.

It is also known that albumin stabilizes drugs and organic compounds such as thromboxane. See Folco, et al. FEBS Letters 83(2):321-324 (1977). In addition, highly purified plasma-derived Factor VIII:C products are known to be stabilized with albumin.

(3) With regard to the use of carbohydrates, Battle et al., Thromb. Haemot. 54(3):697-699 (1985) disclose that infusion of 500 ml of 6% Dextran 70 (70,000 M.W.) into normal adults leads to an apparent decrease in vWF multimers. The maximum effect was at about 6 hours. Factor VIII:C activity also decreased at about 6 hours.

Artusson et al., J. Pharm. Sci. 73(11):1507-1513 (1984), report that biodegradable microparticles of cross-linked starch may be used as carriers of proteins and low molecular weight drugs. The starch used was acrylolated maltodextrin or acrylolated hydroxyethyl starch. The particles were used to immobilize carbonic anhydrase, and it is concluded that the particles may be useful as a drug carrier system.

Aberg et al., Ann. Surg. 189(2):243-247 (1979), describe a study on the effect of an infusion of 500 ml of 6% Dextran 70 on Factor VIII activity. The study is an attempt to clarify the mechanism of the effect of dextran on platelet function. Factor VIII antigen levels significantly decreased after dextran infusion.

Raasch, Am. J. Hosp. Pharm. 36:89-91 (1979), reports that infusion of Dextran 70 may, in some cases, result in reduced Factor VIII activity.

In summary, the above-described prior art discloses the use of various carbohydrates which may be added to final container protein solutions of intermediate purity for stabilization of the protein during heat treatment. Highly purified cell culture derived proteins are stabilized across the lyophilization step with albumin or compounds such as gelatin, glycine, alanine, NaCl, etc. Carbohydrates of high molecular weight may be used as drug carriers.

Accordingly, it is an object of the present invention to provide a compound which may be added as a stabilizer during the normal purification steps of cell culture derived Factor VIII:C, and which will maintain the stability, i.e., molecular integrity and biological activity, of the purified protein.

It is also an object of the invention to provide a stabilizer which is known to be acceptable for intravenous use, but is not derived from a biological (i.e. plasma) source.

It is also an object of the present invention to provide a stabilizer which can be retained across the purification process as part of a final product formulation.

It is a further object of the present invention to provide a stabilized composition comprising factor VIII:C.

The present invention comprises the use of selected carbohydrates for the stabilization of proteins. Thus the above mentioned objects of the present invention can be achieved by providing a composition comprising
(a) therapeutically active recombinant Factor VIII:C having a purity of at least about 60 % total protein;
(b) 1 - 5 % excipients selected from the group consisting of glycine, calcium chloride and sodium chloride; and
(c) about 1 - 50 mg per ml of final container solution of mannitol.

An important advantage in the use of mannitol is that mannitol is expected to be highly suitable for injection into human beings.

Mannitol, M.W. 182, may be represented as follows:

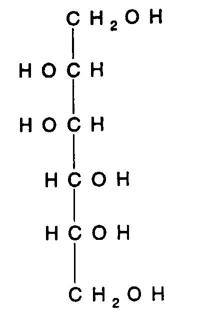

Mannitol is re-added at various process steps but is relatively inexpensive and has high physiological safety and toxicity profiles.

An important aspect of the present invention is that the protein to be stabilized has molecular integrity which is required to produce a measurable biological activity. As the protein to be stabilized the composition of the present invention contains Factor VIII:C, a coagulation factor which is extremely large in its native state (approximately 300 kD) and is extremely labile, being detected in highly purified preparations as a mixture of peptides of 40-210 kD. The integrity of the Factor VIII:C may be assessed by examination of this peptide mixture on gels.

The present invention contemplates the use of 1 - 50 mg/ml, preferably 5 - 10 mg/ml, mannitol as a stabilizer.

### DESCRIPTION OF THE PREFERRED EMBODIMENT

### Materials and Methods

Recombinant Factor VIII:C (rFVIII) was produced from BHK-21 cells transfected with the cDNA coding for human Factor VIII (Vehar et al., Nature 312:337-342 (1984), Wood et al., Nature 312:330-337 (1984)).

Clarified tissue culture fluid was applied to a DEAE-Sepharose column that had been equilibrated, washed and eluted as follows: Equilibration and wash were carried out with 0.02M imidazole, 0.01M CaC1₂, 0.075M NaC1, pH 6.9. Elution was carried out with 0.02M imidazole, 0.01 CaC1₂, 0.25M NaC1, pH 6.9.

Immunoabsorption on a monoclonal antibody (MAb) binding to rFVIII (C7F7) was carried out with immobilized C7F7 antibody. (See Eaton et al., Biochemistry 25(2): 505-512 (1986), EPO 160 457).

The column was equilibrated, washed and eluted as follows: Equilibration and wash were carried out with 0.02M imidazole, 0.01M CaC1₂, 0.5M NaC1, pH 6.9. Elution was carried out with 0.02M imidazole, 1M CaC1₂, 0.7M NaC1, pH 6.9.

C7F7 eluate was applied to a gel filtration column, Pharmacia. Equilibration and wash were carried out with 0.02M imidazole, 0.01M CaC1₂, 0.05M NaC1, .01% Tween 80, pH 6.9.

Diafiltration and ultrafiltration were carried out using an Amicon stirred cell with a YM-30 membrane.

Glycine USP was obtained from Sigma Chemical Corp.; CaC1₂ was obtained from Fisher Scientific; NaCl was obtained from J. T. Baker, USP; albumin was pharmaceutical grade albumin 25%, Plasbumin, Cutter Biological; and sorbitol and mannitol were reagent grade obtained from Baker Chemical Co.

Dextran 70 is defined as that fraction of dextran, a branched polysaccharide composed of glucose units, having an average molecular weight of 70,000. (Physician's Desk Reference, 1989, 43rd ed., p. 1630). Dextran 70 was obtained from Pharmacia.

Factor VIII:C activity was measured by the one stage clotting assay, using the WHO Mega standard. One unit of activity is defined as the coagulation activity of Factor VIII (F.VIII) in one ml of normal plasma. Specific activity is defined as units of activity per total protein mass. Factor VIII may be designated as essentially pure at 5,000 IU/mg specific activity. Purity may also be defined antigenically. F.VIII may be stabilized at greater than about 60% purity by addition of the present carbohydrates.

### Example 1

This Example illustrates a study of various compounds used in stabilizing F.VIII across the freeze-drying step.

F.VIII from clarified tissue culture fluid (tcf) was eluted from a DEAE ion exchange column. A DEAE pool was obtained and the protein was purified through an anti-F.VIII monoclonal antibody designated C7F7 (40 ml column volume). To this eluate was added glycerol to 15%. Then, after G-25 gel filtration desalting (on a 600 ml column), the pool was adjusted to contain 0.275 M glycine. Then, three 45 ml aliquots were taken out. To aliquot number one, albumin was added to 1 mg/ml. This was a control or standard in each set of experiments. The recovery of the F.VIII solution across each step was set at 1.0 based on the albumin control. To the second aliquot, and/or to a third aliquot, another excipient and/or another concentration to be tested was added. These included dextran (M.W. 10,300) sorbitol, mannitol, dextrose, and methyl cellulose. No addition was also a control.

Then each 45 ml aliquot was concentrated 3-fold by ultrafiltration (uf) with a stirred cell and an YM-30 membrane. The 15 ml was then freeze-dried in 5 ml aliquots. Assays taken after concentration and after freeze drying are compared in the table to the albumin control (which is set at 1.0).

The data in Table 1 below indicate that no addition of stabilizer gives almost no recovery, and that mannitol, sorbitol, and dextran all help preserve the Factor VIII activity across the freeze dry step. The stabilizers illustrated compare favorably to albumin. It should also be noted that these results were obtained with the use of 15% glycerol, which is also considered a stabilizer. Results superior to the use of glycerol above were obtained.

**Table 1**

| Dextran | After uf | After freeze-dry |
|---|---|---|
| 50 mg/ml | 1.22 | 1.64 |
| 10 | 2.94 | 1.2 |
| 5 | 1.45 | 1.59 |
| 3 | 1.14 | |
| 2 | 1.5 | 0.81 |
| 1 | 0.91 | 0.61 |
| 1 | 0.64 | 0.33 |
| | | |
| Sorbitol | 0.45 | 0.96 |
| 5 (0 mg/ml) | 0.72 | 0.72 |
| 1 | 0.99 | 0.54 |
| | | |

| Mannitol | | |
|---|---|---|
| 10 mg/ml | 1.14 | 1.28 |
| 5 | 1.19 | 1.46 |
| | | |

| Methyl Cellulose | | |
|---|---|---|
| 10 mg/ml | 1.31 | N.D. |
| 5 | 0.79 | N.D. |
| | | |
| Control no addition | --- | 0.05; 0.3; 0.1 |
| | | |

| Dextran 70 | | |
|---|---|---|
| 5 mg/ml | 1.31 | 2.31 |
| 4 | 1.64 | 2.01 |
| 3 | 1.32 | 2.13 |
| 2 | 1.34 | 1.61 |
| 1 | 0.85 | 1.30 |

### Example 2

This Example utilizes Dextran 70, identified as a freeze-dry stabilizer in the preceding example.

60 ml of C7F7 eluate were obtained after DEAE chromatography of clarified tissue culture fluid, as in the previous example. Glycerol was added to C7F7 eluate to a final concentration of 15%. This was divided into 2 aliquots. To one aliquot albumin was added to a final concentration of 1 mg/ml, to the other Dextran 70 was added to a final concentration of 1 mg/ml. Each aliquot was applied to a 150 ml G-25 gel filtration column as described above to change the buffer and salt concentration to a value which allow F.VIII binding to the next C7F7 column. Each pool from this column was then applied to another 8 ml C7F7 column. The first aliquot produced 13 ml of eluate. 52 µl of 25% albumin was added to yield 1 mg/ml of albumin. The second eluate produced 23 ml of eluate; 442 µl of 6% Dextran 70 was again added for a final dextran concentration of 1 mg/ml. Glycerol was added to each to a final concentration of 15%. Then, a second G-25 column was run to change buffer so that it would be compatible with and bind to the DEAE Sepharose. In the albumin control (first aliquot), 19 ml of eluate resulted. 0.3 ml of 25% albumin was added to achieve a final concentration of 4 mg/ml. Likewise, the pool from the second aliquot had 1.5 ml of 6% Dextran added to a 21.5 ml pool to achieve a final concentration of 4 mg/ml dextran. Each sample was then diafiltered with a YM 30 membrane against 5 volume changes of buffer and adjusted by concentration or dilution to 20 ml. This material was then freeze dried in a standard laboratory freeze-drier (Virtis Unitrap II at -70°C, 0.1 mm Hg).

The results are shown below in Table 2.

**Table 2**

| | Albumin | | | Dextran | | |
|---|---|---|---|---|---|---|
| | u/ml | ml | Yield | u/ml | ml | Yield |
| 1st C7F7 | 216.4 | 30.0 | 100.0%/100.0% | 216.4 | 30.0 | 100.0%/100.0% |
| 1st G-25 | 76.6 | 70.0 | 82.6%/ 82.6% | 88.6 | 64.0 | 87.3%/ 87.3% |
| 2nd C7F7 | 335.3 | 15.0 | 93.8%/ 77.5% | 213.1 | 26.5 | 99.6%/ 87.0% |
| 2nd G-25 | 57.7 | 70.0 | 80.3%/ 62.2% | 62.6 | 59.0 | 65.4%/ 56.9% |
| Overnight | 69.1 | 70.0 | 119.8%/ 74.5% | 76.1 | 59.0 | 121.6%/ 69.2% |
| 3rd DEAE | 178.7 | 19.5 | 72.0%/ 53.7% | 142.4 | 23.0 | 73.0%/ 50.5% |
| Diafilt. | 68.2 | 20.0 | 39.1%/ 21.0% | 71.3 | 20.0 | 43.5%/ 22.0% |
| Freeze-dry | 56.8 | | 83.3%/ 17.5% | 68.7 | | 96.4%/ 21.2% |

Table 2 yields are expressed as step yield/cumulative yield. In each experiment, the stabilizer was at 1 mg/ml during processing, which was increased to 4 mg/ml for freeze drying. Dextran compares favorably with albumin during both processing and freeze-drying.

### Example 3

This example reproduces the experiment of Example 2, but with different stabilizer concentrations.

In this experiment, two 31.5 ml aliquots of 1st C7F7 eluate were obtained, and processed to the 3rd DEAE eluate stage with either dextran or albumin. Then from Aliquot 1, 22 ml of eluate were mixed with .44 ml of 25% albumin to give 5 mg/ml. The Aliquot 2 eluate was mixed with 1.73 ml of 6% dextran (26 ml of eluate) to give 4 mg/ml. Each was then diafiltered vs. 5 volumes of buffer, concentrated to 15 ml each and freeze-dried. The data are shown in Table 3:

**Table 3**

| | Albumin | | | Dextran | | |
|---|---|---|---|---|---|---|
| | u/ml | ml | Yield | u/ml | ml | Yield |
| 1st C7F7 | 114.1 | 31.5 | 100.0%/100.0% | 125.5 | 31.5 | 100.0%/100.0% |
| 1st G-25 | 51.1 | 84.0 | 119.4%/119.4% | 54.9 | 70.0 | 97.2%/ 97.2% |
| 2nd C7F7 | 232.8 | 17.5 | 94.9%/113.4% | 128.9 | 32.0 | 107.3%/104.3% |
| 2nd G-25 | 54.0 | 75.0 | 99.4%/112.7% | 44.1 | 79.0 | 84.5%/ 88.1% |
| Overnight | 51.8 | 75.0 | 95.9%/108.1% | 51.3 | 75.0 | 110.4%/ 97.3% |
| 3rd DEAE | 121.0 | 22.5 | 70.1%/ 75.8% | 120.3 | 27.7 | 86.6%/ 84.3% |
| Diafilt. | 184.6 | 15.0 | 101.7%/ 77.0% | 134.4 | 15.5 | 62.5%/ 52.7% |
| Freeze-dry | 130.0 | | 70.4%/ 54.3% | 99.0 | | 73.7%/ 38.8% |

These data show the comparability of dextran and albumin at 4 mg/ml and 5 mg/ml, respectively.

### Example 4

This Example is directed to final container formulations.

The following Table 4 shows a representative final container formulation wherein albumin has been replaced by dextran. Other excipients in other concentrations can be used. Excipients and stabilizers are adjusted in the final bulk stage, just prior to container filling. The containers are then freeze dried.

**Table 4**

| Ingredient | With albumin | Without albumin |
|---|---|---|
| Factor VIII | 100 u/ml | 100 u/ml |
| Glycine | 0.275M | 0.275M |
| CaCl₂ | 0.0025M | 0.0025M |
| NaCl | 0.1M | 0.1M |
| Albumin | 5-8 mg/ml | 0 |
| Dextran-70 | 0 | 1-5 mg/ml |

Another final container formulation includes, in place of albumin or dextran, mannitol or sorbitol at 5-8 mg/ml. The final container formulation may contain as much as 50 mg/ml of stabilizer.

Mannitol is considered superior to sorbitol for this application because sorbitol is metabolized to produce fructose and may cause fructose intolerance in some patients.

### Example 5

In this series of experiments mannitol was employed in connection with the processing of a second DEAE pool, which was placed on a C7F7 column. To the eluate (10-15 ml), 5 mg/ml mannitol was added. This eluate was applied to a CL6B gel filtration column, pooled, ultrafiltered and diafiltered (UF/DF) and freeze-dried. In some experiments, mannitol was also added to 5 mg/ml to the diafiltration buffer and/or CL6B wash buffer. The results are shown in Table 5 below.

**Table 5**

| | Exp. A | Exp. B | Exp. C |
|---|---|---|---|
| Starting material | 100/100 | 100/100 | 100/100 |
| C7F7 | 81/81* | 124/124* | NA/NA* |
| CL6B | 62/50* | 40/50 | NA/65* |
| UF/DF | 79/40* | 6/3 | 25/16 |
| Freeze-dry | 67/27 | NA | NA |

Results are expressed as percentage step yield/cumulative yield. An asterisk (*) indicates addition of mannitol at that step. Yields in excess of 100% result from assay variability.

The results show that addition of mannitol to the CL6B wash buffer and the uf/df buffer is essential to process yield. Addition of mannitol to additional buffers improves yields, and addition of mannitol to each sequential step dramatically improves yields over the process. The mannitol yield in this series of experiments was comparable to the yield observed in comparable experiments wherein albumin was added at the C7F7 step. Experiments with sorbitol produced results comparable to those with mannitol in Exp. A. Representative yields with sorbitol were: starting material, 100/100; C7F7, 91/91*; CL6B, 86/77*; UF/DF, 88/68*.

### Example 6

A purified murine IgG monoclonal antibody against tumor necrosis factor was studied by HPLC for molecular integrity. All samples were prepared with glycine .27M at pH 4.0.

The following Table shows the molecular size distribution after freezing and thawing. Numbers are % protein in various HPLC peaks, which are denominated high M.W. (aggregates), IgG, and low M.W. (fragments).

**Table 6**

| Sample | High M.W. | IgG | Fragments |
|---|---|---|---|
| Liquid Control | 3 | 97 | - |
| .27 M glycine | 8 | 90 | 2 |
| dextran 5 mg/ml | 3 | 97 | - |
| sorbitol 10 mg/ml | 3 | 97 | - |
| | | | |
| Liquid Control | 2 | 96 | 2 |
| .27 M glycine | 11 | 87 | 2 |
| sorbitol 1 mg/ml | 7 | 91 | 2 |
| sorbitol 5 mg/ml | 4 | 96 | 2 |
| sorbitol 10 mg/ml | 2 | 96 | 2 |
| dextran 1 mg/ml | 9 | 88 | 3 |
| dextran 5 mg/ml | 5 | 92 | 3 |
| mannitol 5 mg/ml | 7 | 90 | 3 |

Liquid controls were not frozen. Highest monomer levels (IgG) were found at dextran 5 mg/ml and sorbitol at 5 or 10 mg/ml. Sorbitol or dextran at 1 mg/ml had little or no protective effect.

It is important to note that all samples contained 0.27M glycine, which has also previously been described as a stabilizer. Significantly superior results were achieved when the compounds of the present invention, were added to samples already containing glycine.

### Discussion

It is well known that highly purified proteins are highly unstable. In these Examples, the rF.VIII used has generally a specific activity of 2,000-5,000 at the immunoabsorption step, corresponding to a purity of roughly 60-99% pure. The stabilizers of the present invention may be used with other highly purified proteins having biological activity, such as monoclonal antibodies. Other purified, biologically active proteins to which the present invention may be applied may include, for example, the following rDNA derived proteins: alpha, beta and gamma interferon, tissue-type plasminogen activator, tumor necrosis factor, human growth hormone, erythropoietin, colony stimulating factors, Factor IX, Protein C, superoxide dismutase, IL-2, epidermal growth factor, etc.

The process steps disclosed herein are merely representative of typical protein purification steps, i.e. anion exchange chromatography, affinity chromatography, gel filtration, ultrafiltration, etc. Other steps such as Protein A chromatography (for purifying IgG's), HPLC, etc. could also be used. No particular significance is attached to the use of the anti-F.VIII:C C7F7 immuno-purification monoclonal antibody used; other MAb's could be used as well.

Among the particular stabilizers used, mannitol is most preferred because it is expected to be well tolerated in human subjects. Dextran or the like may be preferred because its high molecular weight allows it to be carried through the purification process with a high M.W. protein of interest, e.g., F.VIII:C. F.VIII:C generally has a range of molecular weights in its purified form, with 80-90 kD chains being predominant. Thus, the dextran used, with 70 kD M.W. approximately matches the M.W. of the protein chains to be stabilized.

## Claims (Claims for the following Contracting State(s): AT, BE, CH, DE, DK, FR, GB, IT, LI, LU, NL, SE)

1. A composition comprising
(a) therapeutically active recombinant Factor VIII:C having a purity of at least about 60 % total protein;
(b) 1-5 % excipients selected from the group consisting of glycine, calcium chloride and sodium chloride; and
(c) about 1-50 mg per ml of final container solution of mannitol.

2. The composition of claim 1 wherein the Factor VIII:C has a purity of at least about 99 %.

3. The composition of claim 2 wherein the amount of mannitol is about 5-10 mg/ml of final container solution.

4. A method of preparing a composition according to claim 1 comprising the steps:
(A) purifying said Factor VIII:C to at least 60 % of its theoretical maximum specific activity; and
(B) adding a stabilizer which is mannitol in an amount such that the presence of the stabilizer in the final liquid formulation is 1-50 mg/ml for each 100-300 unit/ml of final container Factor VIII:C activity.

5. The method of claim 4 further comprising the step of freeze drying the composition.

6. The method of any of claims 4 and 5 wherein said stabilizer is added to a concentration of 1-5 mg/ml.

7. The method of any of claims 4 and 6 wherein said Factor VIII:C is purified to at least 99% of its theoretical maximum specific activity.

## Claims (Claims for the following Contracting State(s): ES, GR)

1. A method of preparing a composition comprising
(a) therapeutically active recombinant Factor VIII:C having a purity of at least about 60 % total protein;
(b) 1-5 % excipients selected from the group consisting of glycine, calcium chloride and sodium chloride; and
(c) about 1-50 mg per ml of final container solution of mannitol,
comprising the steps:
(A) purifying said Factor VIII:C to at least 60% of its theoretical maximum specific activity; and
(B) adding a stabilizer which is mannitol in an amount such that the presence of the stabilizer in the final liquid formulation is 1-50 mg/ml for each 100-300 unit/ml of final container Factor VIII:C activity.

2. The method of claim 1 further comprising the step of freeze drying the composition.

3. The method of any of claims 1 and 2 wherein said stabilizer is added to a concentration of 1-5 mg/ml.

4. The method of any of claims 1 and 3 wherein said Factor VIII:C is purified to at least 99% of its theoretical maximum specific activity.

## Patentansprüche (Patentansprüche für folgende(n) Vertragsstaat(en): AT, BE, CH, DE, DK, FR, GB, IT, LI, LU, NL, SE)

1. Zusammensetzung, die
a) therapeutisch wirksamen rekombinanten Faktor VIII:C, der eine Reinheit von mindestens etwa 60 % Gesamtprotein hat;
b) 1 bis 5 % Arzneimittelträgersubstanzen, die aus der aus Glycin, Calciumchlorid und Natriumchlorid bestehenden Gruppe ausgewählt sind; und
c) etwa 1 bis 50 mg Mannit pro ml Endbehälterlösung enthält.

2. Zusammensetzung nach Anspruch 1, wobei der Faktor VIII:C eine Reinheit von mindestens etwa 99 % hat.

3. Zusammensetzung nach Anspruch 2, wobei die Mannitmenge etwa 5 bis 10 mg/ml Endbehälterlösung ist.

4. Verfahren zur Herstellung einer Zusammensetzung gemäß Anspruch 1, wobei Stufen enthalten sind, in denen man:
(A) den genannten Faktor VIII:C auf mindestens 60% seiner theoretischen maximalen spezifischen Aktivität reinigt; und
(B) ein Stabilisiermittel zufügt, welches Mannit ist, und zwar in einer solchen Menge, daß die vorhandene Menge des Stabilisiermittels in der endgültigen flüssigen Formulierung 1 bis 50 mg/ml für jede 100 bis 300 Einheiten/ml Endbehälter-Faktor VIII:C-Aktivität beträgt.

5. Verfahren gemäß Anspruch 4,
wobei man ferner die Zusammensetzung gefriertrocknet.

6. Verfahren gemäß jedem der Ansprüche 4 und 5,
wobei das Stabilisiermittel in einer Konzentration von 1 bis 5 mg/ml zugefügt ist.

7. Verfahren gemäß jedem der Ansprüche 4 und 6,
wobei der genannte Faktor VIII:C auf mindestens 99% seiner theoretischen maximalen spezifischen Aktivität gereinigt ist.

## Patentansprüche (Patentansprüche für folgende(n) Vertragsstaat(en): ES, GR)

1. Verfahren zur Herstellung einer Zusammensetzung, die
(a) therapeutisch wirksamen rekombinanten Faktor VIII:C, der eine Reinheit von mindestens etwa 60% Gesamtprotein hat;
(b) 1 bis 5% Arzneimittelträgersubstanzen, die aus der aus Glycin, Calciumchlorid und Natriumchlorid bestehenden Gruppe ausgewählt sind; und
(c) etwa 1 bis 50 mg Mannit pro ml Endbehälterlösung
enthält,
wobei das Verfahren Stufen umfaßt, in denen man:
(A) den genannten Faktor VIII:C auf mindestens 60% seiner theoretischen maximalen spezifischen Aktivität reinigt; und
(B) ein Stabilisiermittel zufügt, welches Mannit ist, und zwar in einer solchen Menge, daß die vorhandene Menge des Stabilisiermittels in der endgültigen flüssigen Formulierung 1 bis 50 mg/ml für jede 100 bis 300 Einheiten/ml Endbehälter-Faktor VIII:C-Aktivität beträgt.

2. Verfahren gemäß Anspruch 1,
wobei man ferner die Zusammensetzung gefriertrocknet.

3. Verfahren gemäß jedem der Ansprüche 1 und 2,
wobei das Stabilisiermittel in einer Konzentration von 1 bis 5 mg/ml zugefügt ist.

4. Verfahren gemäß jedem der Ansprüche 1 und 3,
wobei der genannte Faktor VIII:C auf mindestens 99% seiner theoretischen maximalen spezifischen Aktivität gereinigt ist.

## Revendications (Revendications pour l'(les) Etat(s) contractant(s) suivant(s): AT, BE, CH, DE, DK, FR, GB, IT, LI, LU, NL, SE)

1. Composition comprenant
(a) le facteur VIII:C recombiné thérapeutiquement actif, ayant une pureté d'au moins environ 60% de la protéine totale;
(b) 1-5% d'excipients choisis parmi le groupe consistant en glycine, chlorure de calcium et chlorure de sodium; et
(c) environ 1-50 mg de mannitol par ml de solution finale du conteneur.

2. Composition selon la revendication 1, dans laquelle le facteur VIII:C a une pureté d'au moins environ 99%.

3. Composition selon la revendication 2, dans laquelle la quantité de mannitol est d'environ 5-10 mg/ml de la solution finale de conteneur.

4. Procédé de préparation d'une composition selon la revendication 1 comprenant les étapes de :
(A) purification dudit facteur VIII:C à au moins 60% de son activité spécifique théorique maximum; et
(B) l'addition d'un stabilisant qui est le mannitol dans une quantité telle que la présence du stabilisant dans la formulation finale liquide est de 1-50 mg/ml pour chaque 100-300 unités/ml d'activité finale du facteur VIII:C du conteneur.

5. Procédé selon la revendication 4 comprenant en outre l'étape de lyophilisation de la composition.

6. Procédé selon l'une des revendications 4 et 5 dans lequel ledit stabilisant est ajouté à une concentration de 1-5 mg/ml.

7. Procédé selon l'une des revendications 4 et 5 dans lequel ledit facteur VIII:C est purifié à au moins 99% de son activité spécifique théorique maximum.

## Revendications (Revendications pour l'(les) Etat(s) contractant(s) suivant(s): ES, GR)

1. Procédé de préparation d'une composition comprenant
(a) le facteur VIII:C recombiné thérapeutiquement actif, ayant une pureté d'au moins environ 60% de la protéine totale;
(b) 1-5% d'excipients choisis parmi le groupe consistant en glycine, chlorure de calcium et chlorure de sodium; et
(c) environ 1-50 mg de mannitol par ml de solution finale du conteneur,
comprenant les étapes de :
(A) purification dudit facteur VIII:C à au moins 60% de son activité spécifique théorique maximum; et
(B) l'addition d'un stabilisant qui est le mannitol dans une quantité telle que la présence du stabilisant dans la formulation finale liquide est de 1-50 mg/ml pour chaque 100-300 unités/ml d'activité finale du facteur VIII:C du conteneur.

2. Procédé selon la revendication 1 comprenant en outre l'étape de lyophilisation de la composition.

3. Procédé selon l'une des revendications 1 et 2 dans lequel ledit stabilisant est ajouté à une concentration de 1-5 mg/ml.

4. Procédé selon l'une des revendications 1 et 3 dans lequel ledit facteur VIII:C est purifié à au moins 99% de son activité spécifique théorique maximum.
